(19) Europäisches Patentamt
European Patent Office
Office européen des brevets

(11) Veröffentlichungsnummer: **0 011 734**
**B1**

(12) # EUROPÄISCHE PATENTSCHRIFT

(45) Veröffentlichungstag der Patentschrift: **07.09.83**

(51) Int. Cl.³: **A 61 K 6/00**

(21) Anmeldenummer: **79104333.4**

(22) Anmeldetag: **06.11.79**

(54) **Dentalwerkstoffe auf Basis von organischen Kunststoffen in pastöser Form.**

(30) Priorität: **24.11.78 DE 2850917**

(43) Veröffentlichungstag der Anmeldung: **11.06.80 Patentblatt 80/12**

(45) Bekanntmachung des Hinweises auf die Patenterteilung: **07.09.83 Patentblatt 83/36**

(84) Benannte Vertragsstaaten: **AT BE CH DE FR GB IT LU NL SE**

(56) Entgegenhaltungen:
**DE-A-2 403 211**
**DE-A-2 656 847**
**FR-A-2 028 274**
**FR-A-2 187 826**
**US-A-3 471 596**
**US-A-3 647 498**

(73) Patentinhaber: **BAYER AG, Zentralbereich Patente, Marken und Lizenzen, D-5090 Leverkusen 1, Bayerwerk (DE)**

(72) Erfinder: **Walkowiak, Michael, Dr., Albertus-Magnus-Strasse 10, D-5090 Leverkusen (DE)**
Erfinder: **Podszun, Wolfgang, Dr., Wolfskaul 4, D-5000 Köln 80 (DE)**
Erfinder: **Leusner, Bernhard, Dr., Grüner Weg 148, D-5090 Leverkusen (DE)**
Erfinder: **Süling, Carlhans, Dr., Carl-Leverkus-Strasse 10, D-5074 Odenthal (DE)**
Erfinder: **Schulz, Hans Hermann, Am Neulandkreuz 23, D-5653 Leichlingen (DE)**

**EP 0 011 734 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Dentalwerkstoffe auf Basis von organischen Kunststoffen in pastöser Form

Die Verwendung von gefüllten Kunststoffmaterialien als Werkstoffe für künstliche Zähne, Brücken, Kronen und Zahnfüllungen ist bekannt. Zur Herstellung von Zahnfüllungen werden diese Materialien üblicherweise in Form von Zubereitungen aus anorganischen Füllstoffen, gegebenenfalls organischen Polymerisaten und polymerisierbaren Bindern zur Anwendung gebracht.

Die bisher bekannten Materialien haben auf Grund ihrer Konsistenz und Klebrigkeit anwendungstechnische und klinische Nachteile.

Das Einbringen des Materials in die Kavität erfolgt durch Einstreichen, häufig wird die eingebrachte Masse, bedingt durch das Haften am Füllinstrument, teilweise nach dem Einfüllen in die Kavität von der Kavitätenwandung abgezogen. Diese Erscheinung ist in der Regel vom Zahnarzt nicht feststellbar und führt daher zu nicht wandständigen inkompletten Füllungen mit den bekannten Nachteilen.

Besonders nachteilig wirkt sich das verstärkte Kleben der bisher bekannten Füllmaterialien bei mehrflächigen Kavitäten aus. So ist, wie aus der Amalgamfülltechnik her bekannt, ein einwandfreies Ausfüllen der Kavität nur möglich, wenn ein Füllmaterial portionsweise eingebracht wird. Bei dieser Fülltechnik werden zunächst kleine Portionen wandständig in die Kavitätenwinkel gestopft und anschließend erst die Kavität ausgefüllt. Eine entsprechende Arbeitsweise ist mit den bisher bekannten Kunststoffmaterialien nicht möglich.

Während bei einflächigen Füllungen im Frontzahnbereich die Oberflächengestalt durch Anlegen von Matrizenbändern erzielt wird, bereitet die Formung von okklusalen Flächen mit Materialien, die eine klebende Konsistenz aufweisen, Schwierigkeiten. So war bei den bisher bekannten Materialien eine Formung der Kauflächenbezirke nur in groben Zügen möglich. Nach dem Aushärten war daher üblicherweise eine Formgebung durch rotierende Schleif- und Polierkörper erforderlich. Bekanntlich sind hierbei Verletzungen der benachbarten Schmelzbezirke in der Regel nicht zu vermeiden. Die Folgen hiervon sind Verfälschungen des Kauflächenreliefs und gegebenenfalls okklusale Störungen.

Man hat versucht, die wünschenswerte Oberflächengestalt durch Einstellung einer »schnitzbaren« Eigenschaft zu erzielen. Dieses »schnitzbare« Verhalten tritt jedoch erst dann ein, wenn schon ein gewisser Polymerisationsumsatz erreicht ist. Erfolgt in diesem Zustand die Bearbeitung des Füllmaterials, so kann ein Auf- bzw. Einreißen der Füllungsoberfläche und damit eine Schädigung der Füllung nicht ausgeschlossen werden. Diese Risse, erzielt durch »Schnitzen«, können Eintrittspforten für Microorganismen und für Farbstoffe mit den bekannten Auswirkungen sein. Darüber hinaus kann das Bearbeiten von schon anpolymerisierten Materialien zu Polymerisationsstörungen führen.

Gegenstand der Erfindung sind nun Dentalwerkstoffe auf Basis von organischen Kunststoffen in pastöser Form, enthaltend

a) polymerisierbare Binder,
b) organische Füllstoffe und
c) anorganische Füllstoffe mit einer mittleren Teilchengröße im Bereich von 1 nm bis 1 µm, welche mit einem Haftvermittler nachbehandelt worden sind,

welche dadurch gekennzeichnet sind, daß der organische Füllstoff b) ein vernetztes Perlpolymerisat mit einer mittleren Teilchengröße von 10 bis 100 µ ist und daß der anorganische Füllstoff wenigstens zum Teil im vernetzten Perlpolymerisat enthalten ist.

Dentalmassen in pastöser Form, welche vernetzte Perlpolymerisate mit einem Gehalt an anorganischen Füllstoffen enthalten, sind bisher nicht bekanntgeworden. Es wurde überraschenderweise gefunden, daß diese Pasten sich hervorragend als Zahnfüllmaterial eignen.

In FR-A-2 028 274 wird eine Mischung aus einem vernetzten Perlpolymerisat auf Basis von Methacrylsäureestern, einem polymerisierbaren Binder auf Basis eines Methacrylsäureesters und einem feinteiligen anorganischen Füllstoff beschrieben. Die Teilchen sind jedoch wesentlich größer als gemäß vorliegender Erfindung; im übrigen betrifft diese Literaturstelle Hydrogele, welche als Dentalwerkstoffe unbrauchbar sind. In DE-A-2 403 411 sowie in US-A-3 647 498 werden Dentalwerkstoffe beschrieben, die nicht vernetzte Perlpolymerisate enthalten. Ein Hauptnachteil dieser Produkte ist ihre große Klebrigkeit.

In DE-A-2 656 847 werden Dentalwerkstoffe aus vernetzten Methylmethacrylatpolymeren, anorganischen Füllstoffen und Bindern auf Methacrylatbasis erwähnt. Es handelt sich hierbei jedoch um ein aufgeschlämmtes, nicht pastöses Material; der Literaturstelle ist keinerlei Hinweis auf die erfindungsgemäß erforderliche geringe Teilchengröße sowie den Füllstoff zu entnehmen. Aus US-A-3 471 596 sind pastöse Dentalwerkstoffe bekannt geworden, die polymeres Allylmethacrylat, Methylmethacrylatperlen als Füllstoff und Polyglycol-dimethacrylat sowie gegebenenfalls Pigmente enthalten. Der organische Füllstoff ist in diesem Fall jedoch offensichtlich kein vernetztes Perlpolymer sondern ein Gemisch aus Polymethylmethacrylatpulver und polymerisiertem Allylmethacrylat. Die verwendeten Pigmente sind nicht mit den üblicherweise für Dentalzwecke eingesetzten anorganischen Füllstoffen zu vergleichen, da sich Pigmente sowohl hinsichtlich ihrer chemischen Zusammensetzung als auch hinsichtlich der zu verwendenden Menge von üblichen anorganischen

Füllstoffen unterscheiden. Keiner der zitierten Literaturstellen ist eine Anregung zu entnehmen, anorganische Füllstoffe in ein Perlpolymerisat einzubauen. Diese erfindungsgemäße Maßnahme hat den Vorteil, daß der Füllstoff homogen im auspolymerisierten Dentalmaterial verteilt ist, was unter anderem zu verbesserten mechanischen Werten führt.

Die erfindungsgemäßen Materialien lassen sich darüber hinaus in einer Konsistenz herstellen, die eine Verarbeitung ermöglicht, wie sie in der Amalgamfülltechnik gebräuchlich ist, d. h. sie lassen sich stopfen und modellieren.

Mit den erfindungsgemäßen Materialien gelingt es, auf Grund einer nicht klebenden, festen, stopfbaren Konsistenz, ein- und mehrflächige Kavitäten in mehreren Portionen wandständig aufzufüllen. Durch die besondere Eigenschaft des Materials kommt es beim portionsweisen Stopfen nicht zu Schichtenbildung, d. h. die einzelnen Portionen verbinden sich homogen miteinander.

Nach dem jeweiligen Einbringen einer Portion in die Kavität und dem Stopfen bzw. Adaptieren bleibt diese, ohne ihre Form zu verändern, am Ort liegen, d. h., sie läßt sich auch nicht elastisch deformieren. Die Kavitätenfüllung läßt sich ferner, auf Grund der besonderen Konsistenz, mit sogenannten Amalgampistolen durchführen, ohne daß sich das Füllmaterial wieder von der Kavitätenwandung abzieht oder an der Pistolenöffnung haften bleibt.

Die erfindungsgemäßen Materialien zeigen eine durch Instrumente formbare Konsistenz schon unmittelbar nach dem Anmischvorgang. Diese Konsistenz ermöglicht es, daß nach dem Auffüllen der Kavität, mittels geeigneter Instrumente, z. B. aus Kunststoff oder aus Metall, wie sie in der Amalgamfülltechnik verwendet werden, die okklusale individuelle Kauflächengestalt geformt wird.

Die pastösen erfindungsgemäßen Dentalwerkstoffe auf Basis von organischen Kunststoffen gehen durch Aushärtung in feste Körper über, die den besonderen Vorteil besitzen, gut polierbar zu sein.

Zur Herstellung der erfindungsgemäßen Dentalwerkstoffe werden 20 bis 60, vorzugsweise 25–50 Gew.-Teile polymerisierbare Binder, 20–75, vorzugsweise 30–65 Gew.-Teile der füllstoffhaltigen vernetzten Perpolymerisate, 5–50, vorzugsweise 5–28 Gew.-Teile anorganischer Füllstoffe und 0,01–5 Gew.-Teile Starterzusätze zu einer Paste vermischt.

Zur Erleichterung der Pastenfertigung können Inhibitoren oder Lichtschutzmittel zugesetzt werden. Für bestimmte Indikationen kann es angezeigt sein, zusätzliche Farbstoffe zuzusetzen.

Als polymerisierbare Binder eignen sich die Ester der Methacrylsäure von einwertigen und mehrwertigen Alkoholen gegebenenfalls im Gemisch mit anderen Vinylmonomeren. Besonders günstig ist es, wenn der Gehalt an Methacrylsäureestern über 80% beträgt.

Als geeignete Ester der Methacrylsäure seien beispielsweise aliphatische und cycloaliphatische Ester genannt, wie Methylmethacrylat, Äthylmethacrylat und Cyclohexylmethacrylat.

Besonders gut geeignet sind ferner Ester von mehrwertigen Alkoholen, mit einem Molekulargewicht von 190–10 000, insbesondere Ester von 2- und 3wertigen Alkoholen mit einem Molekulargewicht im Bereich von 190 bis 800, wie z. B. Äthylenglycoldimethacrylat, Triethylenglycoldimethacrylat, Neophentylglycoldimethacrylat oder Trimethylolpropantrimethacrylat, des weiteren Urethan und Ureidpolymethacrylate, die durch Umsetzung von Hydroxyalkylmethacrylaten bzw. Aminoalkylmethacrylaten mit Polyisocyanaten zugänglich sind, z. B. die Verbindung

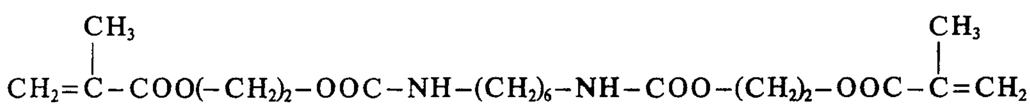

Sehr gute Pasten erhält man, wenn als Binder zumindest in Anteilen Verbindungen vom Typ des Bis-GMA der Formel

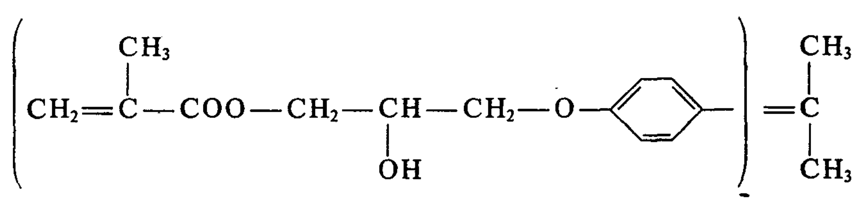

eingesetzt werden.

Zahnfüllmassen mit guter Konsistenz und einem hohen Niveau der mechanischen Festigkeit werden besonders dann erhalten, wenn man als Binder Mischungen aus verschiedenen Methacrylsäureestern verwendet, z. B. Mischungen von 20–70 Gew.-Teilen Bis GMA und 80–30 Gew.-Teilen Triethylenglycoldimethacrylat.

Die zur Pastenherstellung eingesetzten vernetzten Perlpolymerisate sollen zu mehr als 50 Gew.-% aus polymerisierten Methacrylsäureestern, vorzugsweise Methacrylsäuremethylester, bestehen. Als vernetzend wirkende Monomere eignen sich die mit Methylmethacrylat copolymerisierbaren Polyvinylverbindungen, wie beispielsweise Äthylenglycoldimethacrylat, Divinylbenzol, wobei der Vernetzteranteil 2 bis 35 Gew.-% des Monomergemisches betragen sollte. Neben dem Vernetzter können weitere Monomere in das Perlpolymerisat einpolymerisiert sein, um beispielsweise das

Quellverhalten des Perlpolymerisats zu beeinflussen oder um die mechanischen Eigenschaften des ausgehärteten Dentalkunststoffs zu modifizieren. Die mittlere Korngröße der eingesetzten Perlpolymerisate liegt zwischen 10 und 100 µm; besonders günstig ist der Bereich von 15 bis 70 µm.

Die erfindungsgemäß zu verwendenden Polymerperlen enthalten einen anorganischen, feinverteilten Füllstoff. Sie sind vorzugsweise aus polymerisierten (Meth)acrylsäureestern mit einer Viskosität von 0,1 bis 10 Pa · s aufgebaut. Die erfindungsgemäße Verwendung von mit anorganischen Füllstoffen gefüllten Perlpolymerisaten führt zu Dentalwerkstoffen, bei denen sowohl das Perlpolymerisat als auch die Perlzwischenräume gleichermaßen anorganischen Füllstoff enthalten, wodurch ein hohes Maß an Homogenität im ausgehärteten Werkstoff erreicht wird.

Als feinteiliger anorganischer Füllstoff für die erfindungsgemäßen Dentalmaterialien sind in erster Linie Siliziumdioxid, Aluminiumdioxid, Silikate und Silikatgläser geeignet, sofern ihre mittlere Teilchengröße im Bereich von 1 nm – 1 µm liegt. Besonders günstig ist die Verwendung von flammpyrolytisch gewonnenem, amorphem Siliziumdioxyd, und zwar vorzugsweise von amorphem Siliziumdioxid mit einer Primärteilchengröße von 5 – 30 mµ und einer spezifischen Oberfläche gemessen nach BET von 40 – 400 $m^2/g$.

Der feinteilige anorganische Füllstoff wird mit Haftvermittlern, beispielsweise mit Vinyltrimethoxysilan oder Trimethoxy-(3-methacryloyloxypropyl)-silan nachbehandelt, um den Verbund zwischen anorganischem Füllstoff und organischer Matrix zu verbessern.

Als Starterzusätze können die üblichen Startersysteme verwendet werden, d. h. Radikale, Anionen oder Kationen liefernde Systeme, die eine radikalische, anionische oder kationische Polymerisation auslösen können. Im Falle von Radikale liefernden Systemen sind Peroxide oder aliphatische Azoverbindungen besonders geeignet, beispielsweise Benzoylperoxid, Lauroylperoxid oder Azoisobuttersäuredinitril, die üblicherweise in Mengen von 0,1 bis 5 Gew.-% eingesetzt werden. Während die Härtung bei erhöhter Temperatur allein durch Peroxide oder anderer Radikalstarter durchgeführt wird, ist zur Härtung bei Raumtemperatur ein Zusatz von Beschleunigern, vorzugsweise aromatischen Aminen notwendig.

Geeignete Beschleuniger sind N-N-substituierte Toluidine und Xylidine, wie N,N-Dimethyl-p-Toluidin oder N,N-Bis(2-hydroxyethyl)-Xylidin. Gute Aushärtung erzielt man mit 0,5 – 3% Aminzusatz. Eine günstige Darbietungsform für ein Peroxid/Beschleuniger aktiviertes System ist die 2-Pasten-Form, wobei eine Paste den Radikalstarter und die andere den Beschleuniger enthält und die Aushärtung durch Mischen beider Pasten eingeleitet wird.

Auch eine Aushärtung unter Verwendung von UV-Licht oder sichtbarem Licht bei geeigneter Sensibilisierung ist eine sehr gute Methode. Geeignete Photoinitiatoren sind beispielsweise Benzophenon und seine Derivate, Benzoin und seine Derivate, wie Benzoinäther, Anthrachinone und aromatische Disulfide.

### Beispiel 1

### Herstellung eines mit amorphem Siliziumdioxid gefüllten Perlpolymerisats

#### Polymerisation

Reaktionsgefäß:
6-Liter-Autoklav mit Doppelankerrührer

Lösung I:
2500 ml dest. Wasser

(Dispergatorlösung):
500 ml 7,5%ige wäßrige Lösung des Copolymerisats aus 1 Gew.-Teil Methacrylsäure und 1 Gew.-Teil Methylmethacrylat mit dem pH = 6 und der Viskosität: 3650 mPas

Lösung II:

| | |
|---|---|
| 684 g | Methylmethacrylat |
| 36 g | Ethylenglycoldimethacrylat |
| 308 g | silanisiertes amorphes Siliziumdioxid (BET-Oberfläche 170 $m^2/g$) |
| 7,2 g | Benzoylperoxid |

Lösung I wird vorgelegt und 5 Minuten verrührt. Bei stehendem Rührer gibt man Lösung II auf einmal zu und bespült den Autoklaven mit Stickstoff. Anschließend wird 5 bar Stickstoff aufgedrückt, die Rührergeschwindigkeit auf 400 UpM eingestellt und auf 80° C aufgeheizt. Bei einsetzender exothermer Reaktion wird so stark gekühlt, daß die Temperatur unter 90° C bleibt. Man läßt 2 Stunden bei 80° C nachrühren.

Aufarbeitung

Der Ansatz wird abgeblasen und mit dest. Wasser auf 10 l verdünnt. Nach der Zugabe von 180 g Eisessig wird 15 Minuten auf 90—100°C aufgeheizt. Das ausfallende Perlpolymerisat wird nach Erkalten abgesaugt, durch dreimaliges Aufrühren in je 5 l dest. Wasser gewaschen und bei 60°C getrocknet.

Ausbeute:

866 g Perlpolymerisat
mittlerer Perldurchmesser: 45 µm.

Beispiel 2

A) Peroxidpaste

110 g Perlpolymerisat aus Beispiel 1,
28 g silanisiertes amorphes Siliziumdioxid (BET-Oberfläche 170 $m^2/g$)
47 g Bis-GMA (Nupol 46-4005 der Firma Freeman Chemical)
25 g Triäthylenglycoldimethacrylat
1,5 g Bis-o-toluyl-peroxid

Die einzelnen Komponenten werden in einen Kneter gegeben und 60 Minuten lang intensiv geknetet, wobei während der letzten 10 Minuten ein Vakuum von ca. 20 Torr angelegt wird. Man erhält auf diese Weise eine knetbare Masse von besonders fester Konsistenz.

B) Aminpaste

Perlpolymerisat, Bis-GMA und Triäthylenglycoldimethacrylat werden in den gleichen Mengen wie bei der Peroxidpaste (A) eingesetzt und verarbeitet. Anstelle des Peroxids werden jedoch 1,2 g N,N-Bis-(2-Hydroxypropyl)-3,5-dimethylanilin eingesetzt.

C) Pastöse Masse zum Füllen von Zähnen

Gleiche Teile (z. B. je 200 mg) der Amin- und Peroxidpaste werden 30 Sekunden lang intensiv gemischt. Die erhaltene Mischung ist in hervorragender Weise als Zahnfüllmaterial geeignet. Sie härtet in wenigen Minuten unter geringem Polymerisationsschrumpf aus.

**Patentansprüche**

1. Dentalwerkstoffe auf Basis von organischen Kunststoffen in pastöser Form, enthaltend

a) polymerisierbare Binder,
b) organische Füllstoffe und
c) anorganische Füllstoffe mit einer mittleren Teilchengröße im Bereich von 1 nm bis 1 µm, welche mit einem Haftvermittler nachbehandelt worden sind,

dadurch gekennzeichnet, daß der organische Füllstoff b) ein vernetztes Perlpolymerisat mit einer mittleren Teilchengröße von 10 bis 100 µm ist und daß der anorganische Füllstoff wenigstens zum Teil im vernetzten Perlpolymerisat enthalten ist.

2. Dentalwerkstoffe nach Anspruch 1, dadurch gekennzeichnet, daß die polymerisierbaren Binder zu mehr als 80 Gew.-% aus Estern der Methacrylsäure bestehen.

3. Dentalwerkstoffe nach Anspruch 1 oder 2, dadurch gekennzeichnet, daß die polymerisierbaren Binder zu mindestens 50 Gew.-% aus Estern der Methacrylsäure mit zwei oder mehr polymerisierbaren Doppelbindungen bestehen.

4. Dentalwerkstoffe nach Anspruch 1 bis 3, dadurch gekennzeichnet, daß die vernetzten Perlpolymerisate zu mehr als 50 Gew.-% aus Estern der Methacrylsäure aufgebaut sind.

5. Dentalwerkstoffe nach Anspruch 1 bis 4, dadurch gekennzeichnet, daß die anorganischen Füllstoffe aus Siliciumdioxid, Aluminiumdioxid, Silicaten oder Silicatgläsern bestehen.

6. Dentalwerkstoffe nach Anspruch 1 bis 5, dadurch gekennzeichnet, daß die anorganischen

Füllstoffe aus Kieselsäure bestehen.

7. Dentalwerkstoffe nach Anspruch 1 bis 6, dadurch gekennzeichnet, daß die anorganischen Füllstoffe silanisiert sind.

8. Dentalwerkstoffe nach Anspruch 1 bis 7, dadurch gekennzeichnet, daß der Anteil an vernetzten Perlpolymerisaten und anorganischen Füllstoffen zusammen 52–80 Gew.-% beträgt.

9. Dentalwerkstoffe nach Anspruch 1 bis 8, dadurch gekennzeichnet, daß der Anteil an anorganischen Füllstoffen 5–50 Gew.-% beträgt.

10. Dentalwerkstoffe nach Anspruch 1 bis 9, dadurch gekennzeichnet, daß der Anteil an anorganischen Füllstoffen 5–28 Gew.-% beträgt.

## Claims

1. Dental materials based on organic plastics in paste form, containing

a) polymerisable binders,
b) organic fillers and
c) inorganic fillers which have an average particle size in the range of 1 nm to 1 μm and have been after-treated with an adhesion promoter,

characterised in that the organic filler b) is a crosslinked bead polymer having an average particle size of 10 to 100 μm and in that at least part of the inorganic filler is contained in the crosslinked bead polymer.

2. Dental materials according to Claim 1, characterised in that the polymerisable binders consist to the extent of more than 80% by weight of esters of methacrylic acid.

3. Dental materials according to Claim 1 or 2, characterised in that the polymerisable binders consist to the extent of at least 50% by weight of esters of methacrylic acid with two or more polymerisable double bonds.

4. Dental materials according to Claim 1 to 3, characterised in that the crosslinked bead polymers are made up to the extent of more than 50% by weight of esters of methacrylic acid.

5. Dental materials according to Claim 1 to 4, characterised in that the inorganic fillers consist of silicon dioxide, aluminium dioxide, silicates or silicate glasses.

6. Dental materials according to Claim 1 to 5, characterised in that the inorganic fillers consist of silicic acid.

7. Dental materials according to Claim 1 to 6, characterised in that the inorganic fillers are silanised.

8. Dental materials according to Claim 1 to 7, characterised in that the proportion of crosslinked bead polymers and inorganic fillers together is 52–80% by weight.

9. Dental materials according to Claim 1 to 8, characterised in that the proportion of inorganic fillers is 5–50% by weight.

10. Dental materials according to Claim 1 to 9, characterised in that the proportion of inorganic fillers is 5–28% by weight.

## Revendications

1. Produits dentaires à base de matières organiques synthétiques sous la forme de pâte, contenant

a) des liants polymérisables,
b) des charges organiques et
c) des charges inorganiques en particules d'une grosseur moyenne comprise dans la plage de 1 nm à 1 μm, qui ont été exposées à un traitement subséquent avec un agent de pontage,

caractérisés en ce que le charge organique b) est un produit réticulé de polymérisation en perles ayant un diamètre moyen de particules de 10 à 100 μm et en ce que la charge inorganique est contenue au moins en partie dans le produit réticulé de polymérisation en perles.

2. Produits dentaires suivant la revendication 1, caractérisés en ce que les liants polymérisables se composent à plus de 80% en poids d'esters de l'acide méthacrylique.

3. Produits dentaires suivant la revendication 1 ou 2, caractérisés en ce que les liants polymérisables se composent à au moins 50% en poids d'esters de l'acide méthacrylique présentant deux ou plus de deux doubles liaisons polymérisables.

4. Produits dentaires suivant les revendications 1 à 3, caractérisés en ce que les produits réticulés de polymérisation en perles sont formés à plus de 50% en poids d'esters d'acide méthacrylique.

5. Produits dentaires suivant les revendications 1 à 4, caractérisés en ce que les charges inorganiques sont formées de dioxyde de silicium, de dioxyde d'aluminium, de silicates ou de verres au silicate.

6. Produits dentaires suivant les revendications 1 à 5, caractérisés en ce que les charges inorganiques sont formées d'acide silicique.

7. Produits dentaires suivant les revendications 1 à 6, caractérisés en ce que les charges inorganiques sont traitées avec un silane.

8. Produits dentaires suivant les revendications 1 à 7, caractérisés en ce que les proportions de produits réticulés de polymérisation en perles et de charges inorganiques forment ensemble 52 à 80% en poids.

9. Produits dentaires suivant les revendications 1 à 8, caractérisés en ce que la proportion de charges inorganiques s'élève à 5—50% en poids.

10. Produits dentaires suivant les revendications 1 à 9, caractérisés en ce que la proportion de charges inorganiques s'élève à 5—28% en poids.